Europäisches Patentamt

European Patent Office

Office européen des brevets

(11) Veröffentlichungsnummer: **0 167 146**
**B1**

(12) **EUROPÄISCHE PATENTSCHRIFT**

(45) Veröffentlichungstag der Patentschrift: **24.10.90**

(51) Int. Cl.⁵: **C 07 H 17/08, A 61 K 31/70**

(21) Anmeldenummer: **85108176.0**

(22) Anmeldetag: **02.07.85**

(54) **Primycin-salze, Verfahren zur deren Herstellung sowie diese enthaltende pharmazeutische Präparate.**

(30) Priorität: **03.07.84 HU 257184**

(43) Veröffentlichungstag der Anmeldung:
**08.01.86 Patentblatt 86/02**

(45) Bekanntmachung des Hinweises auf die
Patenterteilung:
**24.10.90 Patentblatt 90/43**

(84) Benannte Vertragsstaaten:
**AT BE CH DE FR GB IT LI NL**

(56) Entgegenhaltungen:
FR-A-2 431 508

"THE MERCK INDEX", 9. Ausgabe, 1976, Seite
1004, Merck & Co., Inc., Rahway, N.J., US;

(73) Patentinhaber: **CHINOIN Gyogyszer és
Vegyészeti Termékek Gyára RT.
To utca 1-5
H-1045 Budapest IV (HU)**

(72) Erfinder: **Dékany, Gyula
Ipar utca 2
H-1095 Budapest (HU)**
Erfinder: **Frank, Judit
Szöllö köz 2
H-1032 Budapest (HU)**

(74) Vertreter: **Bartsch, Elisabeth, Dr.
Patentanwälte Lotterhos & Partner
Lichtensteinstrasse 3
D-6000 Frankfurt am Main 1 (DE)**

Courier Press, Leamington Spa, England.

**Beschreibung**

Die Erfindung betrifft neue Primycin-salze, Verfahren zu deren Herstellung, sowie diese enthaltende pharmazeutische Zubereitungen.

Das Primycin ist ein bekanntes Antibioticum von makrolidem Typ (Nature *174*, 1105, 1954). Die Herstellung von Primycin durch Fermentierung von Streptomyces primycini und die Isolierung in Form des Sulfats ist aus der HU—PS 146 332 bekannt. Die HU—PS 151 197 betrifft eine Weiterentwicklung der Aufarbeitung, die HU—PS 153 593 eine Fermentierung von Thermopolyspora galeriensis und die HU—PS 179 148 eine modifizierte Aufarbeitung, wobei zunächst das Primycinsulfat hergestellt wird, aus dem dann mit Alkali die Primycinbase freigesetzt wird. Aus der so erhaltenen Base werden dann mit Mineralsäuren — außer Schwefelsäure — die verschiedenen Salze hergestellt.

Die neuesten, empfindlichen dünnschichtchromatographischen Untersuchungen haben jedoch gezeigt, daß die alkalische Behandlung von Primycinsulfat zur Veränderung der Struktur der Primycinkomponente führt, und daß infolgedessen die Struktur der Primycinsalze, die aus der so erhaltenen Primycinbase hergestellt worden sind, nicht mehr mit der des Ausgangsmaterials identisch ist.

Ziel der Erfindung war, ein neues Verfahren zur Herstellung von Primycin-salzen zu entwickeln, wobei Primycinsulfat ohne alkalische Behandlung in industriellem Maßstab in die verschiedenen Primycinsalze übergeführt werden kann, so daß die Feinstruktur und die antibiotische Wirkung des Primycins unverändert erhalten bleibt.

Es wurde gefunden, daß sich Primycinsulfat durch Umsetzung mit Bariumsalzen unmittelbar und ohne Anwendung von Alkali in die verschiedenen Primycin-salze überführen läßt. Das dabei gebildete Bariumsulfat läßt sich aus der Reaktionsmischung leicht durch Filtration entfernen, so daß die gebildeten Primycin-salze in einfacher Weise in sehr reiner Form isoliert werden können.

Die Erfindung betrifft neue Primycinsalze, deren Primycinkomponente in Struktur und antibiotischer Wirkung gegenüber dem durch Fermentation erhaltenen Primycin unverändert ist, und die als Säurekomponente den Rest von organischen Säuren mit 1—16 C-Atomen, vorzugsweise aliphatischen $C_{1-4}$ bzw. $C_{14-16}$-Carbonsäuren, halogenierten aliphatischen Carbonsäuren, aliphatischen Dicarbonsäuren, aromatischen Carbonsäuren, substituierten aromatischen Carbonsäuren, organischen Sulfonsäuren oder von anorganischen Säuren, mit Ausnahme der Schwefelsäure, vorzugsweise Halogensäuren, enthalten. Einige Beispiele hierfür sind: Primycin-formiat, Primycin-acetat, Primycin-chloracetat, Primycin-trichloracetat, Primycin-propionat, Primycin-palmitat, Primycin-oxalat, Primycin-perchlorat, Primycin-bromid, Primycin-jodid, Primycin-benzoat, Primycin-mesylat sowie Primycin-tosylat.

Die Erfindung betrifft auch ein Verfahren zur Herstellung der neuen Primycin-salze, wobei Primycinsulfat mit Bariumsalzen umgesetzt wird, die aus Bariumcarbonat oder Bariumhydroxid und den entsprechenden salzbildenden Reagenzien erhalten werden. Als salzbildende Reagenzien können organische oder anorganische Säuren verwendet werden, deren Bariumsalze in Wasser und/oder in organischen Lösungsmitteln, vorzugsweise in aliphatischen $C_{1-4}$-Alkoholen löslich sind.

Bevorzugte salzbildende Reagenzien sind organische Säuren, die 1—16 C-Atome enthalten, insbesondere aliphatische $C_{1-4}$- bzw. $C_{14-16}$-Carbonsäuren, z.B. Ameisensäure, Essigsäure, Palmitinsäure, halogenierte aliphatische Carbonsäuren, z.B. Trichloressigsäure, Monochloressigsäure etc., aliphatische Dicarbonsäuren, z.B. Oxalsäure, aromatische Carbonsäuren, z.B. Benzoesäure, substituierte aromatische Carbonsäuren, z.B. Pikrinsäure, organische Sulfonsäuren, z.B. p-Toluol-sulfonsäure, Methansulfonsäure, oder anorganische Säuren, insbesondere Halogensäure, z.B. Halogenwasserstoffsäure, wie Bromwasserstoff, Jodwasserstoff oder Perchlorsäure.

Unter aliphatischen Carbonsäuren sind solche mit 1—4 bzw. 14—16 C-Atomen, unter halogenierten aliphatischen Carbonsäuren sind solche mit 1—4 C-Atomen, die durch ein bis drei Halogene, vorzugsweise Chlor, substituiert sind, und unter gegebenenfalls substituierten aromatischen Carbonsäuren sind vorzugsweise Benzoesäure, Phthalsäure, die durch ein oder mehrere Halogene, vorzugsweie Chlor, Nitrogruppen und/oder $C_{1-4}$-Alkylgruppen substituiert sein können, zu verstehen.

Die Bariumsalze können auch in situ gebildet werden, indem das Bariumcarbonat entweder in den salzbildenden Reagenzien oder in aliphatischen $C_{1-4}$-Alkoholen, vorzugsweise in Methanol, gelöst und dann das salzbildende Reagens zugesetzt wird. Die erhaltene Lösung wird im Vakuum eingedampft und der so erhaltene Rückstand in Äther oder aliphatischem $C_{1-4}$-Alkohol, vorzugsweise Methanol, gelöst.

Nach einer anderen Variante zur Bariumsalzherstellung wird vom Bariumhydroxid ausgegangen und die gesättigte wäßrige Bariumhydroxidlösung mit einer Lösung der salzbildenden Reagenzien in aliphatischen $C_{1-4}$-Alkoholen, vorzugsweise Methanol, und/oder Wasser vereinigt und das so erhaltene Bariumsalz durch Filtration abgetrennt.

Zur Durchführung des Verfahrens gemäß Erfindung wird zweckmäßig eine Suspension von Primycinsulfat in aliphatischen $C_{1-4}$-Alkoholen, vorzugsweise Methanol, mit dem entsprechenden Bariumsalz bei einer Temperatur im Bereich von 20—80°C, vorzugsweise bei Siedetemperatur des Lösungsmittels, umgesetzt, und das ausgefallene Bariumsulfat durch Filtration entfernt. Die Reaktion wird vorzugsweise beim Siedepunkt der Reaktionsmischung durchgeführt, um eine vollständige Umsetzung und gleichzeitig das Ausfällen des Bariumsulfats in leicht filtrierbarer Form zu erreichen. Das Bariumsulfat wird bei Siedetemperatur, vorzugsweise unter Verwendung von Filterfilfsstoffen, wie Celite, abfiltriert. Das Filtrat wird im Vakuum eingedampft und der so erhaltene feste Rückstand mit Äther verrieben, abfiltriert, mit Äther und/oder einer Mischung von Aceton und Wasser (2:1) gewaschen und getrocknet. Die verschiedene Primycin-salze werden in Form eines Pulvers erhalten.

Die neuen Primycin-salze der Erfindung weisen eine bessere Löslichkeit und bessere Absorption als das bislang bekannte Primycinsulfat auf.

Die antibakterielle Wirkung der neuen Primycin-salze der Erfindung ist bei polyresistenten grampositiven und gramnegativen Bakterien, sowie bei Sproßpilzen untersucht worden. Die Ergebnisse zeigen, daß die neuen Primycin-salze eine hervorragende Wirkung bei grampositiven Bakterien und eine gemäßigte Wirking bei gramnegativen Bakterien und bei Sproßpilzen aufweisen.

Die Untersuchungen wurden bei Bakterien auf Difco-Bouillon-Nährboden und bei Pilzen auf Sabouiraud-Nährboden durchgeführt. Die Inokulation erfolgte bei einer Zellkonzentration von $5 \times 10^5 - 5 \times 10^6$ pro ml und die Inkubation bei einer Temperatur von 37°C für 24 Stunden. Die MIC-Werte (MIC = minimal inhibitory concentration) wurden durch Verdünnung bestimmt und in µg/ml Einheiten angegeben. Es wurde eine Stammlösung mit einer Konzentration von 100 µg/ml in einer Mischung von Butanol, Äthanol und Wasser (1:1:2) hergestellt und dann mit destilliertem Wasser verdünnt. Die untersuchten Bakterien und Pilze sind in Tabelle 1 und die erhaltenen MIC-Werte in Tabelle 2 angegeben.

Untersuchte Primycin-salze:

I. Primycin-acetat
II. Primycin-formiat
III. Primycin-propionat
IV. Primycin-trichloracetat
V. Primycin-perchlorat
VI. Primycin-oxalat
VII. Primycin-palmitat
VIII. Primycin-tosylat
IX. Primycin-benzoat
X. Primycin-jodid
XI. Primycin-bromid

## Tabelle 1

Untersuchte Stämme:

| | | |
|---|---|---|
| 1. | Staphylococcus aureus | CCM.885 |
| 2. | Staphylococcus aureus | DSM.20231 |
| 3. | Staphylococcus aureus | CCM.2317 |
| 4. | Staphylococcus aureus | CCM.2326 |
| 5. | Staphylococcus aureus | CCM.2514 |
| 6. | Staphylococcus aureus | CCM.2515 |
| 7. | Staphylococcus epidermis | CCM.2271 |
| 8. | Staphylococcus aureus Smith | |
| 9. | Streptococcus faecalis | CCM.1875 |
| 10. | Streptococcus agalactiae | CCM.5153 |
| 11. | Streptococcus agalactiae | CCM.5534 |
| 12. | Streptococcus disgalactiae | CCM.5548 |
| 13. | Bacillus subtilis | ATCC.6633 |
| 14. | Bacillus cereus | CCM.2010 |
| 15. | Bacillus licheniformis | CCM.2182 |
| 16. | Bacillus licheniformis | CCM.2205 |
| 17. | Bacillus subtilis | CCM.1718 |
| 18. | Listeria monocytogenes | CCM.5576 |
| 19. | Micrococcus flavus | ATCC.10240 |
| 20. | Micrococcus luteus | DSM.20030 |
| 21. | Sporosarcina ureae | DSM.317 |
| 22. | Pseudomonas aeruginosa | CCM.1960 |
| 23. | Proteus vulgaris | CCM.1799 |
| 24. | Shigella sonnei | CCM.1373 |
| 25. | Salmonella typhimurium | CCM.5445 |
| 26. | Saccharomyces cerevisiae | OKI.1282 |
| 27. | Candida albicans | CBS.562 |
| 28. | Candida tropicalis | CBS.433 |

## Tabelle 2

Die Wirkung von Primycin-salzen auf polyresistente Bakterien und Sproßpilze

| Untersuch-te Stämme | I | II | III | IV | V | VI | VII | VIII | IX | X | XI |
|---|---|---|---|---|---|---|---|---|---|---|---|
| 1 | 0,25 | 0,1 | 0,1 | 0,1 | 0,1 | 0,05 | 0,05 | 0,25 | 0,25 | 0,075 | 0,075 |
| 2 | 0,075 | 0,05 | 0,075 | 0,05 | 0,075 | 0,05 | 0,05 | 0,1 | 0,25 | 0,075 | 0,075 |
| 3 | 0,05 | 0,075 | 0,075 | 0,05 | 0,075 | 0,05 | 0,05 | 0,1 | 0,25 | 0,075 | 0,075 |
| 4 | 0,25 | 0,1 | 0,25 | 0,075 | 0,1 | 0,05 | 0,05 | 0,25 | 0,25 | 0,075 | 0,075 |
| 5 | 0,075 | 0,075 | 0,05 | 0,05 | 0,05 | 0,025 | 0,025 | 0,1 | 0,25 | 0,05 | 0,05 |
| 6 | 0,25 | 0,05 | 0,025 | 0,05 | 0,025 | 0,025 | 0,01 | 0,05 | 0,25 | 0,05 | 0,05 |
| 7 | 0,075 | 0,1 | 0,075 | 0,05 | 0,05 | 0,025 | 0,025 | 0,075 | 0,25 | 0,075 | 0,075 |
| 8 | 0,1 | 0,075 | 0,075 | 0,075 | 0,075 | 0,05 | 0,05 | 0,25 | 0,25 | 0,05 | 0,075 |
| 9 | 2,5 | 2,5 | 5 | 2,5 | 2,5 | 1 | 10 | 2,5 | 5 | 2,5 | 5 |
| 10 | 0,25 | 0,075 | 0,5 | 0,25 | 0,1 | 0,075 | 0,075 | 0,25 | 0,25 | 0,25 | 0,25 |
| 11 | 0,5 | 0,5 | 0,5 | 0,5 | 0,5 | 0,1 | 0,25 | 0,75 | 0,75 | 0,5 | 0,5 |
| 12 | 0,75 | 0,75 | 0,75 | 0,75 | 1 | 0,5 | 0,75 | 1 | 1 | 0,75 | 0,75 |
| 13 | 0,075 | 0,05 | 0,05 | 0,075 | 0,075 | 0,025 | 0,025 | 0,075 | 0,25 | 0,075 | 0,075 |
| 14 | 0,1 | 0,05 | 0,1 | 0,075 | 0,075 | 0,05 | 0,05 | 0,1 | 0,25 | 0,075 | 0,05 |
| 15 | 0,25 | 0,075 | 0,075 | 0,075 | 0,075 | 0,05 | 0,075 | 0,1 | 0,25 | 0,075 | 0,075 |
| 16 | 0,1 | 0,05 | 0,05 | 0,075 | 0,05 | 0,05 | 0,05 | 0,075 | 0,25 | 0,05 | 0,05 |
| 17 | 0,25 | 0,05 | 0,05 | 0,05 | 0,05 | 0,05 | 0,05 | 0,05 | 0,25 | 0,05 | 0,025 |
| 18 | 0,75 | 0,25 | 0,25 | 0,25 | 0,25 | 0,1 | 0,25 | 0,25 | 0,25 | 0,5 | 0,25 |

EP 0 167 146 B1

Fortsetzung von Tabelle 2

| Untersuch-te Stämme | Primycin-salze | | | | | | | | | | |
| | I | II | III | IV | V | VI | VII | VIII | IX | X | XI |
|---|---|---|---|---|---|---|---|---|---|---|---|
| 19 | 0,075 | 0,025 | 0,025 | 0,025 | 0,05 | 0,025 | 0,025 | 0,05 | 0,25 | 0,025 | 0,025 |
| 20 | 0,025 | 0,025 | 0,025 | 0,025 | 0,025 | 0,025 | 0,05 | 0,05 | 0,5 | 0,025 | 0,05 |
| 21 | 0,01 | 0,025 | 0,01 | 0,01 | 0,025 | 0,025 | 0,1 | 0,025 | 0,5 | 0,01 | 0,01 |
| 22 | 25 | 25 | 25 | 25 | 25 | 25 | 25 | 25 | 25 | 25 | 25 |
| 23 | 25 | 25 | 10 | 25 | 25 | 25 | 25 | 25 | 25 | 25 | 25 |
| 24 | 25 | 25 | 10 | 25 | 25 | 25 | 25 | 25 | 25 | 25 | 25 |
| 25 | 25 | 25 | 25 | 25 | 25 | 25 | 25 | 25 | 25 | 25 | 25 |
| 26 | 25 | 25 | 25 | 25 | 25 | 25 | 25 | 25 | 25 | 10 | 50 |
| 27 | 25 | 25 | 25 | 10 | 10 | 10 | 25 | 25 | 25 | 25 | 25 |
| 28 | 10 | 25 | 25 | 10 | 25 | 25 | 25 | 25 | 25 | 10 | 25 |

## EP 0 167 146 B1

Die Erfindung betrifft auch pharmazeutische Präparate, die als Wirkstoffe ein oder mehrere der neuen Primycin-salze mit einer organischen oder anorganischen Säure — ausgenommen Schwefelsäure — und gewünschtenfalls eine oder mehrere weitere antimikrobiell wirkende Substanzen sowie geeignete inerte pharmazeutische Trägermaterialien, Füllstoffe und/oder Hilfsstoffe enthalten. Der Gehalt an Primycin-salzen und gegebenenfalls an weiteren antimikrobiellen Wirkstoffen in derartigen Präparaten beträgt 0,001—10%.

Die Primycin-salze der Erfindung können allein oder gegebenenfalls mit anderen pharmazeutischen Wirkstoffen vermischt in pharmazeutische Zubereitungen mit antibiotischer Wirkung überführt werden, z.B. in feste Formulierungen, wie Tabletten, Kapseln, Dragées und Suppositorien, in halbfeste Formulierungen, wie Salben und Gele, oder in flüssige Formulierungen, wie injizierbare Lösungen, Suspensionen oder Syrupe. Bevorzugte Formulierungen sind Gele, Salben, Puder, injizierbare Lösungen oder Suspensionen, sowie Pulverampulle-Lösungsmittelampulle-Kombinationen.

Die pharmazeutischen Zubereitungen der Erfindung sind für die orale, parenterale oder rektale bzw. lokale Applikation bestimmt. Sie können neben den Wirkstoffen übliche pharmazeutische Trägermaterialien, wie z.B. Magnesiumcarbonat, Magnesiumstearat, Stärke, Talkum, Wasser etc. oder Cyclodextrin als neuen Trägerstoff, sowie gegebenenfalls weitere Hilfsstoffe, wie Zerfallsstoffe, Emulgatoren u.ä. enthalten.

Zur oralen Verabreichung werden Tabletten, Kapseln oder Dragées, zur parenteralen Verabreichung wäßrige Emulsionen, Lösungen oder Suspensionen und zur lokalen Applikation Puder, Salben, wäßrige oder ölige Emulsionen oder durch Befeuchten imprägnierte Verbandstoffe, sowie Suspensionen und Sprays verwendet.

Die Präparate der Erfindung können auch in der Veterinärmedizin verwendet werden, z.B. durch Vermischung in Form von Lösungen mit dem Futter.

Die Erfindung wird durch folgende Beispiele näher erläutert. Hieraus sind keine Beschränkungen herzuleiten.

### Beispiel 1

0,088 g (0,446 mMol) Bariumcarbonat erhitzt man in 5 ml Ameisensäure bis zur vollständigen Lösung zum Sieden, dampft die farblose Lösung im Vakuum zur Trockene ein, löst den Rückstand in 10 ml Methanol und setzt die so erhaltenen Lösung einer Suspension von 1,0 g (0,887 mMol) Primycinsulfat in 70 ml Methanol zu. Man erhitzt die erhaltene Mischung 10 Minuten unter ständigem Rühren zum Sieden, saugt das ausgefallene Bariumsulfat mit Celite heiß ab, engt das Filtrat im Vakuum ein und verreibt den Rückstand mit Äther. Das so erhaltene weiße Pulver wäscht man mit 20 ml einer Mischung von Aceton und Wasser (2:1). Man erhält 0,96 g (94,8%) Primycinformiat, Fp.: 162—164°C, $[\alpha]_D^{25}$ = +31,9°. Die optische Drehung wurde in einer Mischung aus n-Butanol, Äthanol und Wasser (2:1:1) bei einer Konzentration von 0,1% gemessen.

### Beispiel 2

0,88 g (4,459 mMol) wasserfreies Bariumcarbonat rührt man in 10 ml Essigsäure bei einer Temperatur von 100°C bis zur vollständigen Lösung, entfernt das Lösungsmittel im Vakuum, löst den Rückstand in 20 ml Methanol und gibt ihn in eine Suspension von 10 g (8,87 mMol) Primycinsulfat in 60 ml Methanol. Die erhaltene Suspension erhitzt man 10 Minuten unter ständigem Rühren zum Sieden, saugt das ausgefallene Bariumsulfat mit Celite ab und dampft das Filtrat unter vermindertem Druck ein. Den so erhaltenen Rückstand verreibt man mit 50 ml einer Mischung von Aceton und Wasser (2:1) und saugt ab. Nach Trocknung erhält man 9,40 g (91,43%) Primycin-acetat in Form einer weißen Pulvers. Fp.: 186—188°C, $[\alpha]_D^{25}$ = +30°.

### Beispiel 3

Eine Lösung von 0,088 g (0,446 mMol) wasserfreiem Bariumcarbonat und 0,146 g (0,887 mMol) Trichloressigsäure in 20 ml Methanol erhitzt man bis zur vollständigen Lösung des Bariumcarbonats zum Sieden, gibt die erhaltene heiße farblose Lösung in eine Suspension von 1,0 g (0,887 mMol) Primycinsulfat in 80 ml Methanol und erhitzt die Mischung 20 Minuten zum Sieden. Dann saugt man das ausgefallene Bariumsulfat heiß ab, dampft das Filtrat im Vakuum ein, verreibt den Rückstand in Äther, saugt ab und wäscht mit 15 ml einer Mischung von Aceton und Wasser (2:1). Man erhält 0,96 g (85,9%) Primycin-trichloracetat, Fp: 165—166°C, $[\alpha]_D^{25}$ = +17,7°.

### Beispiel 4

Man rührt 0,088 g (0,446 mMol) Bariumcarbonat in 5 ml Propionsäure bei einer Temperatur von 100°C bis zur vollständigen Lösung, dampft die erhaltene farblose Lösung bei vermindertem Druck zur Trockene ein, löst den Rückstand in 10 ml Methanol und gibt ihn zu einer Suspension von 1,0 g (0,887 mMol) Primycinsulfat in 60 ml Methanol. Die Suspension erhitzt man 10 Minuten unter ständigem Rühren zum Sieden, saugt das ausgefallene Bariumsulfat mit Celite heiß ab und dampft das Filtrat im Vakuum ein. Man verreibt den Rückstand mit 20 ml einer Mischung von Aceton und Wasser (2:1) und saugt ab. Man erhält 0,95 g (91,3%) sandfarbenes Primycin-propionat, Fp.: 170°C, $[\alpha]_D^{25}$ = +36,9°.

### Beispiel 5

Man löst 0,22 g (0,887 mMol) Palmitinsäure in 15 ml Methanol und fügt tropfenweise so viel Wasser zu, daß die Palmitinsäure in Lösung bleibt. Zu dieser Lösung gibt man 2,51 ml einer gesättigten Bariumhydroxidlösung, wobei das entstehende Bariumsalz ausfällt, das man absaugt, und zu einer Suspension von 1,0 g (0,887 mMol) Primycinsulfat in 120 ml Methanol gibt. Nach einer Stunde Erhitzen zum Sieden ist das Bariumpalmitat in Lösung gegangen und unlösliches Bariumsulfat fällt aus, das man heiß mit Celite absaugt. Das Filtrat dampft man bei vermindertem Druck ein, verreibt den Rückstand mit Äther und saugt ihn ab, wäscht ihn mit einer Mischung von Aceton und Wasser (2:1) und trocknet ihn bis zur Gewichtskonstanz. Man erhält 0,98 g (82,2%) Primycin-palmitat, Fp.: 180—181°C, $[\alpha]_D^{25} = +30°$.

### Beispiel 6

Man suspendiert 0,087 g (0,444 mMol) Bariumcarbonat in 15 ml Methanol, setzt 0,04 g (0,444 mMol) Oxalsäure zu, erhitzt die erhaltene Suspension bis zur vollständigen Lösung (etwa 10 Minuten) zum Sieden und entfernt das Lösungsmittel unter vermindertem Druck. Den Rückstand gibt man zu einer Lösung von 1,0 g (0,887 mMol) Primycinsulfat in 80 ml Methanol und erhitzt 20 Minuten unter ständigem Rühren zum Sieden. Dann saugt man das ausgefallene Bariumsulfat mit Celite heiß ab, dampft das Filtrat unter vermindertem Druck bis zur Trockene ein, verreibt den Rückstand mit Äther, saugt ab und wäscht mit einer Mischung von Aceton und Wasser (2:1). Man erhält 0,93 g (92,2%) weißes Primycin-oxalat, Fp.: 186—187°C, $[\alpha]_D^{25} = +40.0°$.

### Beispiel 7

Man löst 0,087 g (0,444 mMol) Bariumcarbonat in 8,87 ml 0,1 n wäßriger Perchlorsäure unter milden Erwärmen und entfernt das Wasser im Vakuum. Den Rückstand nimmt man in 10 ml Methanol auf und gibt ihn zu einer Suspension von 1,0 g (0,887 mMol) Primycinsulfat in 80 ml Methanol. Die erhaltene Mischung erhitzt man 20 Minuten unter Rühren zum Sieden, wobei das gebildete Primycin-perchlorat in Lösung bleibt, während das Bariumsulfat ausfällt. Man saugt die heiße Lösung mit Celite ab, und wäscht mit 10 ml einer Mischung von Aceton und Wasser (2:1). Man erhält 0,94 g (88,7%) weißes Primycin-perchlorat, Fp.: 170—171°C, $[\alpha]_D^{25} = +20,0°$.

### Beispiel 8

Man löst 0,087 g (0,444 mMol) Bariumcarbonat in 1 ml 48%igem Bromwasserstoff unter mildem Erwärmen, entfernt das Lösungsmittel unter vermindertem Druck und verreibt den Rückstand mit Äther. Dann saugt man das Bariumbromid ab und wäscht es zur Entfernung von Spuren von Brom mit Dichlormethan. Das gelbe Bariumbromid verfärbt sich nach beige. Danach löst man es in 10 ml Methanol, gibt es zu einer Lösung von 1,0 g (0,887 mMol) Primycinsulfat in 80 ml Methanol und erhitzt die erhaltene Suspension 20 Minuten unter Rühren zum Sieden. Dann saugt man das ausgefallene Bariumsulfat ab, dampft das Filtrat unter vermindertem Druck zur Trockene ein, verreibt den Rückstand mit Äther, saugt ab und wäscht zunächst mit 10 ml einer Mischung von Aceton und Wasser (2:1) und danach mit 10 ml Dichlormethan. Man erhält 0,91 g (87,2%) hellbeiges Primycin-bromid, Fp.: 138°C, $[\alpha]_D^{25} = -20°$.

### Beispiel 9

Man löst 0,087 g (0,444 mMol) Bariumcarbonat in 1,5 ml 57%igem Jodwasserstoff in Methanol unter mildem Erwärmen, entfernt das Lösungsmittel unter vermindertem Druck, verreibt den Rückstand mit Äther und wäscht mit 30 ml Dichlormethan. Das gelbe Bariumjodid löst man in 15 ml Methanol und setzt es einer Suspension von 1,0 g (0,887 mMol) Primycinsulfat in 80 ml Methanol zu. Die Suspension erhitzt man 20 Minuten unter Rühren bis zur vollständigen Lösung des Primycinjodids zum Sieden, saugt den Niederschlag mit Celite heiß ab und dampft das Filtrat unter vermindertem Druck ein. Den Rückstand verreibt man mit Äther, saugt ab und wäscht zunächst mit 10 ml einer Mischung von Aceton und Wasser (2:1) und dann mit Dichlormethan bis zum jodfreien Zustand. Man erhält 0,93 g (85,7%) gelbes Primycin-jodid, Fp.: 178°C, $[\alpha]_D^{25} = +10°$.

### Beispiel 10

0,087 g (0,444 mMol) Bariumcarbonat suspendiert man in 15 ml Methanol, gibt 0,11 g (0,91 mMol) Benzoesäure zu und erhitzt die Suspension bis zur vollständigen Lösung zum Sieden. Die erhaltene farblose Lösung setzt man einer Suspension von 1,0 g (0,887 mMol) Primycinsulfat in 80 ml Methanol zu, erhitzt 20 Minuten und ständigem Rühren zum Sieden, saugt die Reaktionsmischung mit Celite heiß ab und dampft das Filtrat unter vermindertem Druck ein. Den Rückstand verreibt man mit Äther, saugt ab und wäscht mit 15 ml einer Mischung von Aceton und Wasser (2:1). Man erhält 0,98 g (90,6%) Primycin-benzoat in Form eines weißen Pulvers, Fp.: 170—171°C, $[\alpha]_D^{25} = +36,0°$.

### Beispiel 11

Man erhitzt 0,088 g (0,446 mMol) Bariumcarbonat und 0,169 g (0,892 mMol) p-Toluolsulfonsäure-Monohydrat in 20 ml Methanol bis zur vollständigen Lösung zum Sieden, gibt die farblose, heiße Lösung zu einer Suspension von 1,0 g (0,887 mMol) Primycinsulfat in 80 ml Methanol und erhitzt die Mischung 15 Minuten unter ständigem Rühren zum Sieden. Dann saugt man mit Celite heiß ab und dampft das Filtrat unter vermindertem Druck ein. Den Rückstand suspendiert man in einer Mischung von Aceton und Wasser (2:1), saugt ab und wäscht mit der obigen Mischung. Man erhält 1,05 g (93,3%) weißes Primycin-tosylat, das unter Zersetzung schmilzt, $[\alpha]_D^{25} = -3,9°$.

Beispiel 12

Man löst 0,088 g (0,446 mMol) Bariumcarbonat und 0,21 g (2,23 mMol) Monochloressigsäure in 10 ml einer Mischung von Methanol und Wasser (1:1) unter Erwärmen, fügt die heiße Lösung einer Suspension von 1,0 g (0,887 mMol) Primycinsulfat in 70 ml Methanol zu, erhitzt die Mischung 10 Minuten unter ständigem Rühren zum Sieden, saugt dann das ausgefallene Bariumsulfat mit Celite heiß ab und dampft das Filtrat im Vakuum ein. Man verreibt den Rückstand mit Äther, saugt ab, wäscht dreimal mit je 10 ml wassergesättigtem Äther und trocknet bis zur Gewichtskonstanz. Man erhält 0,97 g (93,3%) Primycin-monochloracetat, Fp.: 176°C, $[\alpha]_D^{25} = +22,0°$.

Die nachfolgenden Beispiele erläutern die Zusammensetzung einiger Zubereitungen (Präparate) der Erfindung.

Beispiel 13

*Puder I*

| | |
|---|---|
| Primycin-salz | 1,0 g |
| Cyclodextrin | 9,0 g |
| | 10,0 g |

Beispiel 14

*Puder II*

| | |
|---|---|
| Primycin-salz | 1,0 g |
| Amylum non mucilaginosum | 9,0 g |
| | 10,0 g |

Beispiel 15

*Puder III*

| | |
|---|---|
| Primycin-salz | 1,0 g |
| Äther-oleum lavandulae | 0,02 g |
| Acid. silicium colloidale | 0,10 g |
| Magnesium stearinicum | 0,10 g |
| Zincum oxidatum | 0,20 g |
| Bolus alba | 0,50 g |
| Magnesium carbonicum hydroxydatum | 1,0 g |
| Cyclodextrin | 7,08 g |
| | 10,0 g |

### Beispiel 16

*Aerosolpuder*

| | |
|---|---|
| Primycin-salz | 0,20 g |
| Isopropylmyristat | 1,0 g |
| Freon 11/12 5050 | 98,80 g |
| | 100,0 g |

### Beispiel 17

*Wasseraerosol*

| | |
|---|---|
| Primycin-salz | 0,5 g |
| Äthanol, 62,5% | 10,0 g |
| Wasser | 39,5 g |
| Treibgas | 50,0 g |
| | 100,0 g |

### Beispiel 18

*Aerosol-Wundverschluß*

| | |
|---|---|
| Primycin-salz | 0,1 g |
| Polyvinylpyrrolidon | 1,50 g |
| Äthanol, 62,5% | 48,40 g |
| Treibgas | 50,0 g |
| | 100,0 g |

### Beispiel 19

*Gel I*

| | |
|---|---|
| Primycin-salz | 0,02 g |
| Chlorophyll | 0,002 g |
| Menthol | 0,2 g |
| Polyadipinsäure | 0,12 g |
| Triäthanolamin | 0,15 g |
| Tween 20 | 0,15 g |
| Diisopropyladipinat | 0,50 g |
| Äthanol, 96% | 5,00 g |
| Aqua dest, ad . | 10,0 g |

Beispiel 20

*Gel II*

| | |
|---|---:|
| Primycin-salz | 0,20 g |
| Lidocain | 2,00 g |
| Chlorophyll | 0,005 g |
| Menthol | 0,20 g |
| Carbopol 940 | 1,20 g |
| Triäthanolamin | 1,50 g |
| Tween 20 | 1,50 g |
| Isodiapat | 5,00 g |
| Äthanol, 96% | 50,00 g |
| Aqua dest, ad | 100,00 g |

Beispiel 21

*Salbe*

| | |
|---|---:|
| Primycin-salz | 2,00 g |
| Oleum Paraffini | 33,00 g |
| Vaselinum album | 31,00 g |
| Äthanol, 96% | 20,00 g |
| Tween 60 | 9,00 g |
| Wollfett | 5,00 g |
| | 100,00 g |

Beispiel 22

*Augensalbe*

| | |
|---|---:|
| Primycin-salz | 0,04 g |
| Äthanol, 62,5% | 0,08 g |
| Aqua dest. | 3,16 g |
| Bienenwachs | 300,00 g |
| Cholesterin | 25,00 g |
| Öl ad | 1000,00 g |

11

### Beispiel 23

*Mit Wasser abwaschbare Salbe I*

| | |
|---|---|
| Primycin-salz | 2,00 g |
| Tween 60 | 5,00 g |
| flüssiges Paraffin | 5,00 g |
| Cetylstearylalkohol | 15,00 g |
| weiße Vaseline | 25,00 g |
| Aqua dest. ad | 1000,00 g |

### Beispiel 24

*Mit Wasser abwaschbare Salbe II*

| | |
|---|---|
| Primycin-salz | 0,100 g |
| Äthanol, 62,5% | 2,000 g |
| Wasser | 7,900 g |
| Sorbaxäthen stearat (Tween 60) | 3,600 g |
| flüssiges Paraffin | 3,600 g |
| Cetylstearylalkohol | 10,800 g |
| weiße Vaseline | 18,000 g |
| Propyl-p-oxy-benzoat | 0,054 g |
| Methyl-p-oxy-benzoat | 0,126 g |
| Äthanol, 96% | 2,930 g |
| Lidocainum-Chlorid | 1,000 g |
| Aqua dest. ad | 1000,000 g |

### Beispiel 25

*Augentropfen*

| | |
|---|---|
| Primycin-salz | 0,02 g |
| Natriumhydrogencarbonat | 18,00 g |
| viskoses Lösungsmittel (4 g Methylcellulose + 3,5 g NaCl + 510 g Aqua dest. ad) | 510,00 g |
| Phenyl-mercuri-borat, 0,1% | 15,1 g |
| Aqua dest. ad | 1000,00 g |

Beispiel 26

*Augentropfen (auf Ölbasis)*

| | |
|---|---|
| Primycin-salz | 0,02 g |
| Äthanol, 62,5% | 0,40 g |
| Aqua dest. ad | 1,58 g |
| Cholesterin | 25,00 g |
| steriles Ricinusöl ad | 100,00 g |

Beispiel 27

*Suppositorium vaginalii*

| | |
|---|---|
| Primycin-salz | 0,02 g |
| Äthanol, 62,5% | 0,262 g |
| Gelatine | 1,40 g |
| Natriumacetat | 0,26 g |
| Glycerin | 4,50 g |
| Aqua dest. ad | 9,50 g |

Beispiel 28

*Tablette solubilis*

| | |
|---|---|
| Primycin-salz | 0,4 g |
| Harnstoff oder Natriumchlorid | 9,6 g |

Man löst das Primycin-salz und den Füllstoff in 60%igem Äthanol, dampft das Lösungsmittel ein und tablettiert das Granulat.

Beispiel 29

*Antimikrobieller Verbandstoff*

Man löst das Primycin-salz vorzugsweise in Äthanol und imprägniert den Verbandstoff, z.B. die Mullbinde, mit dieser Lösung, sterilisiert den Verbandstoff und führt die weitere Bearbeitung in an sich bekannter Weise durch.

Der erhaltene antimikrobielle Verbandstoff kann in beliebiger Zeit verwendet werden.

**Patentansprüche für die Vertragsstaaten: BE CH DE FR GB IT LI NL**

1. Neue Primycinsalze, deren Primycinkomponente in Struktur und antibiotischer Wirkung gegenüber dem durch Fermentation erhaltenen Primycin unverändert ist, und die als Säurekomponente den Rest von organischen Säuren mit 1—16 C-Atomen, vorzugsweise aliphatischen $C_{1-4}$ bzw. $C_{14-16}$-Carbonsäuren, halogenierten aliphatischen Carbonsäuren, aliphatischen Dicarbonsäuren, aromatischen Carbonsäuren, substituierten aromatischen Carbonsäuren, organischen Sulfonsäuren oder von anorganischen Säuren, mit Ausnahme der Schwefelsäure, vorzugsweise Halogensäuren, enthalten.

2. Primycin-salze nach Anspruch 1, die als Säurekomponente den Formiat-, Acetat-, Monochloracetat-, Trichloracetat-, Propionat-, Palmitat-, Oxalat-, Perchlorat-, Bromid-, Jodid-, Benzoat-, Mesylat- oder Tosylatrest enthalten.

3. Verfahren zur Herstellung von Primycinsalzen, deren Primycinkomponente in Struktur und antibiotischer Wirkung gegenüber dem durch Fermentation erhaltenen Primycin unverändert ist, dadurch gekennzeichnet, daß man eine Suspension von Primycinsulfat in einem aliphatischen $C_{1-4}$-Alkohol mit einem Bariumsalz einer organischen Säure oder einer anorganischen Säure umsetzt und das erhaltene Primycin-salz isoliert.

4. Verfahren nach Anspruch 3, dadurch gekennzeichnet, daß man zur Umsetzung ein Bariumsalz einer aliphatischen $C_{1-4}$- bzw. $C_{14-16}$ Carbonsäure, einer halogenierten aliphatischen Carbonsäure, einer aliphatischen Dicarbonsäure, einer aromatischen Carbonsäure, einer substituierten aromatischen Carbonsäure, einer organischen Sulfonsäure oder einer Halogensäure verwendet.

5. Verfahren nach Ansprüchen 3 und 4, dadurch gekennzeichnet, daß man als Bariumsalz ein solches verwendet, das in situ gebildet wird.

6. Verfahren nach Ansprüchen 3 bis 5, dadurch gekennzeichnet, daß man zur in situ-Herstellung der verwendeten Bariumsalze Bariumcarbonat oder dessen Lösung in einem aliphatischen $C_{1-4}$-Alkohol in der entsprechenden organischen oder anorganischen Säure löst, die so erhaltene Lösung eindampft und den Rückstand in einem aliphatischen $C_{1-4}$-Alkohol oder in Äther löst.

7. Verfahren nach Ansprüchen 3 bis 5, dadurch gekennzeichnet, daß man zur in situ-Herstellung der verwendeten Bariumsalze eine wäßrige Lösung von Bariumhydroxid mit einer Lösung der entsprechenden organischen oder anorganischen Säure in einem aliphatischen $C_{1-4}$-Alkohol und/oder Wasser umsetzt und abfiltriert.

8. Verfahren nach Ansprüchen 3 bis 7, dadurch gekennzeichnet, daß man die Umsetzung von Primycinsulfat mit einem Bariumsalz bei einer Temperatur im Bereich von 20—80°C, vorzugsweise bei Siedetemperatur des Lösungsmittels, durchführt.

9. Verfahren nach Ansprüchen 3 bis 8, dadurch gekennzeichnet, daß man zur Isolierung des Primycinsalzes das Bariumsulfat abfiltriert, das Filtrat im Vakuum eindampft und den Rückstand mit einem Lösungsmittel, vorzugsweise mit einer Mischung von Aceton und Wasser (2:1) und/oder Äther, verreibt.

10. Pharmazeutische Präparate mit antibiotischer Wirkung, die als Wirkstoff ein oder mehrere Primycin-salze der Ansprüche 1 und 2 und gegebenenfalls weitere antimikrobielle Stoffe enthalten.

**Patentansprüche für den Vertragsstaat: AT**

1. Verfahren zur Herstellung von Primycinsalzen, deren Primycinkomponente in Struktur und antibiotischer Wirkung gegenüber dem durch Fermentation erhaltenen Primycin unverändert ist, dadurch gekennzeichnet, daß man eine Suspension von Primycinsulfat in einem aliphatischen $C_{1-4}$-Alkohol mit einem Bariumsalz einer organischen Säure oder einer anorganischen Säure umsetzt und das erhaltene Primycinsalz isoliert.

2. Verfahren nach Anspruch 1, dadurch gekennzeichnet, daß man zur Umsetzung ein Bariumsalz einer aliphatischen $C_{1-4}$- bzw. $C_{14-16}$-Carbonsäure, einer halogenierten aliphatischen Carbonsäure, einer aliphatischen Dicarbonsäure, einer aromatischen Carbonsäure, einer substituierten aromatischen Carbonsäure, einer organischen Sulfonsäure oder einer Halogensäure verwendet.

3. Verfahren nach Anspruch 2, dadurch gekennzeichnet, daß man als Bariumsalz Bariumformiat, -acetat, -monochloracetat, -trichloracetat, -propionat, -palmitat, -oxalat, -perchlorat, -bromid, -jodid, -benzoat, -mesylat oder -tosylat verwendet.

4. Verfahren nach Ansprüchen 1 bis 3, dadurch gekennzeichnet, daß man als Bariumsalz ein solches verwendet, das in situ gebildet wird.

5. Verfahren nach Ansprüchen 1 bis 4, dadurch gekennzeichnet, daß man zur in situ-Herstellung der verwendeten Bariumsalze Bariumcarbonat oder dessen Lösung in einem aliphatischen $C_{1-4}$-Alkohol in der entsprechenden organischen oder anorganischen Säure löst, die so erhaltene Lösung eindampft und den Rückstand in einem aliphatischen $C_{1-4}$-Alkohol oder in Äther löst.

6. Verfahren nach Ansprüchen 1 bis 5, dadurch gekennzeichnet, daß man zur in situ-Herstellung der verwendeten Bariumsalze eine wäßrige Lösung von Bariumhydroxid mit einer Lösung der entsprechenden organischen oder anorganischen Säure in einem aliphatischen $C_{1-4}$-Alkohol und/oder Wasser umsetzt und abfiltriert.

7. Verfahren nach Ansprüchen 1 bis 6, dadurch gekennzeichnet, daß man die Umsetzung von Primycinsulfat mit einem Bariumsalz bei einer Temperatur im Bereich von 20—80°C, vorzugsweise bei Siedetemperatur des Lösungsmittels, durchführt.

8. Verfahren nach Ansprüchen 1 bis 7, dadurch gekennzeichnet, daß man zur Isolierung des Primycinsalzes das Bariumsulfat abfiltriert, das Filtrat im Vakuum eindampft und den Rückstand mit einem Lösungsmittel, vorzugsweise mit einer Mischung von Aceton und Wasser (2:1) und/oder Äther, verreibt.

9. Verwendung von einem oder mehreren der nach den Verfahren der Ansprüche 1 bis 8 erhältlichen Primycin-salze, gegenbenenfalls zusammen mit weiteren antibakteriellen Wirkstoffen, als Wirkstoffe zur Herstellung von pharmazeutischen Präparaten mit antibiotischer Wirkung.

**Revendications pour les Etats contractants: BE CH DE FR GB IT LI NL**

1. Nouveaux sels de Primycine, dont la Primycine composante a une structure et une activité antibiotique non modifiées par rapport à celles de la Primycine obtenue par fermentation, et qui contiennent en tant que composant acide le radical d'un acide organique en C 1—C 16, de préférence un acide carboxylique aliphatique en C 1—C 4 ou en C 14—C 16, d'un acide carboxylique aliphatique halogéné, d'un acide dicarboxylique aliphatique, d'un acide carboxylique aromatique, d'un acide carboxylique aromatique substitué, d'un acide organique sulfonique ou d'un acide minéral, à l'exception de l'acide sulfurique, de préférence d'un acide halogéné.

2. Sels de Primycine selon la revendication 1, contenant en tant que composant acide le radical formiate, acétate, monochloracétate, trichloracétate, propionate, palmitate, oxalate, perchlorate, bromure, iodure, benzoate, méthane-sulfonate ou toluène-sulfonate.

3. Procédé de préparation de sels de Primycine dont la Primycine composante a une structure et une activité antibiotique non modifiées par rapport à celles de la Primycine obtenue par fermentation, caractérisé en ce que l'on fait réagir une suspension de sulfate de Primycine dans un alcool aliphatique en C 1—C 4 avec un sel de baryum d'un acide organique ou minéral et on isole le sel de Primycine obtenu.

4. Procédé selon la revendication 3, caractérisé en ce que l'on utilise pour la réaction un sel de baryum d'un acide aliphatique carboxylique en C 1—C 4 ou en C 14—C 16, d'un acide carboxylique aliphatique halogéné, d'un acide dicarboxylique aliphatique, d'un acide carboxylique aromatique, d'un acide carboxylique aromatique substitué, d'un acide organique sulfonique ou d'un acide halogéné.

5. Procédé selon les revendications 3 et 4, caractérisé en ce que l'on utilise un sel de baryum formé in situ.

6. Procédé selon les revendications 3 à 5, caractérisé en ce que, pour la préparation in situ du sel de baryum mis en oeuvre, on dissout le carbonate de baryum, tel quel ou à l'état de solution dans un alcool aliphatique en C 1—C 4, dans l'acide organique ou minéral correspondant, on évapore la solution obtenue et on redissout le résidu dans un alcool aliphatique en C 1—C 4 ou dans l'éther.

7. Procédé selon les revendications 3 à 5, caractérisé en ce que, pour la préparation in situ du sel de baryum mis en oeuvre, on fait réagir une solution aqueuse d'hydroxyde de baryum avec une solution de l'acide organique ou minéral correspondant dans un alcool aliphatique en C 1—C 4 et/ou l'eau, et on filtre.

8. Procédé selon les revendications 3 à 7, caractérisé en ce que la réaction du sulfate de Primycine avec un sel de baryum est effectuée à une température dans l'intervalle de 20 à 80°C, de préférence à la température d'ébullition du solvant.

9. Procédé selon les revendications 3 à 8, caractérisé en ce que, pour isoler le sel de Primycine, on filtre le sulfate de baryum, on évapore le filtrat sous vide et on triture le résidu avec un solvant, de préférence avec un mélange acétone/eau, 2:1, et/ou avec l'éther.

10. Compositions pharmaceutiques à activité antibiotique contenant un ou plusieurs sels de Primycine des revendications 1 et 2 en tant que substances actives, avec, le cas échéant, d'autres substances antimicrobiennes.

**Revendications pour l'Etat contractant: AT**

1. Procédé de préparation de sels de Primycine dont la Primycine composante a une structure et une activité antibiotique non modifiées par rapport à celles de la Primycine obtenue par fermentation, caractérisé en ce que l'on fait réagir une suspension de sulfate de Primycine dans un alcool aliphatique en C 1—C 4 avec un sel de baryum d'acide organique ou minéral et on isole le sel de Primycine obtenu.

2. Procédé selon la revendication 1, caractérisé en ce que l'on utilise pour la réaction un sel de baryum d'un acide carboxylique aliphatique en C 1—C 4 ou en C 14—C 16, d'un acide carboxylique aliphatique halogéné, d'un acide dicarboxylique aliphatique, d'un acide carboxylique aromatique, d'un acide carboxylique aromatique substitué, d'un acide organique sulfonique ou d'un acide halogéné.

3. Procédé selon la revendication 2, caractérisé en ce que l'on utilise en tant que sel de baryum le formiate, l'acétate, le monochloracétate, le trichloracétate, le propionate, le palmitate, l'oxalate, le perchlorate, le bromure, l'iodure, le benzoate, le méthane-sulfonate ou le toluène-sulfonate de baryum.

4. Procédé selon les revendications 1 à 3, caractérisé en ce que l'on utilise un sel de baryum formé in situ.

5. Procédé selon les revendications 1 à 4, caractérisé en ce que, pour la préparation in situ du sel de baryum mis en oeuvre, on dissout le carbonate de baryum, tel quel ou à l'état de solution dans un alcool aliphatique en C 1—C 4, dans l'acide organique ou minéral correspondant, on évapore la solution obtenue et on redissout le résidu dans un alcool aliphatique en C 1—C 4 ou dans l'éther.

6. Procédé selon les revendications 1 à 5, caractérisé en ce que, pour la préparation in situ du sel de baryum mis en oeuvre, on fait réagir une solution aqueuse d'hydroxyde de baryum avec une solution de l'acide organique ou minéral correspondant dans un alcool aliphatique en C 1—C 4 et/ou l'eau et on filtre.

7. Procédé selon les revendications 1 à 6, caractérisé en ce que la réaction du sulfate de Primycine avec un sel de baryum est effectuée à une température dans l'intervalle de 20 à 80°C, de préférence à la température d'ébullition du solvant.

8. Procédé selon les revendications 1 à 7, caractérisé en ce que, pour isoler le sel de Primycine, on filtre le sulfate de baryum, on évapore le filtrat sous vide et on triture le résidu avec un solvant, de préférence un mélange d'acétone et d'eau (2:1) et/ou de l'éther.

9. Utilisation d'un ou plusieurs des sels de Primycine obtenus par les procédés des revendications 1 à 8, éventuellement avec d'autres substances actives antibactériennes, en tant que substances actives pour la préparation de compositions pharmaceutiques à activité antibiotique.

**Claims for the Contracting States: BE CH DE FR GB IT LI NL**

1. New primycin salts, in which the primycin component is unchanged in structure and antibiotic activity from the primycin obtained by fermentation, and which contain as acid component the radical of organic acids having 1 to 16 carbon atoms, preferably aliphatic $C_{1-4}$ or $C_{14-16}$ carboxylic acids, halogenated aliphatic carboxylic acids, aliphatic dicarboxylic acids, aromatic carboxylic acids, substituted aromatic carboxylic acids, organic sulphonic acids or of inorganic acids, with the exception of sulphuric acid, preferably halic acids.

2. Primycin salts according to claim 1, which contain as acid component the formate, acetate, monochloroacetate, trichloroacetate, propionate, palmitate, oxalate, perchlorate, bromide, iodide, benzoate, mesylate or tosylate radical.

3. Process for preparing primycin salts the primycin component of which is unchanged in its structure and antibiotic activity from the primycin obtained by fermentation, characterised in that a suspension of primycin sulphate in an aliphatic $C_{1-4}$ alcohol is reacted with a barium salt of an organic acid or an inorganic acid and the resulting primycin salt is isolated.

4. Process according to claim 3, characterised in that a barium salt of an aliphatic $C_{1-4}$ or $C_{14-16}$ carboxylic acid, a halogenated aliphatic carboxylic acid, an aliphatic dicarboxylic acid, an aromatic carboxylic acid, a substituted aromatic carboxylic acid, an organic sulphonic or a halic acid is used in the reaction.

5. Process according to claims 3 and 4, characterised in that the barium salt used is one which is formed *in situ*.

6. Process according to claims 3 to 5, characterised in that, in order to prepare *in situ* the barium salts used, barium carbonate or a solution thereof in an aliphatic $C_{1-4}$ alcohol is dissolved in the corresponding organic or inorganic acid, the resulting solution is concentrated by evaporation and the residue is dissolved in an aliphatic $C_{1-4}$ alcohol or in ether.

7. Process according to claims 3 to 5, characterised in that, in order to prepare *in situ* the barium salts used, an aqueous solution of barium hydroxide is reacted with a solution of corresponding organic or inorganic acid in an aliphatic $C_{1-4}$ alcohol and/or water and then filtered off.

8. Process according to claims 3 to 7, characterised in that the reaction of primycin sulphate with a barium salt is carried out at a temperature in the range from 20 to 80°C, preferably at the boiling temperature of the solvent.

9. Process according to claims 3 to 8, characterised in that, in order to isolate the primycin salt, the barium sulphate is filtered off, the filtrate is concentrated by evaporation *in vacuo* and the residue is triturated with a solvent, preferably with a mixture of acetone and water (2:1) and/or ether.

10. Pharmaceutical preparations having an antibiotic activity which contain as active substance one or more primycin salts according to claims 1 and 2 and optionally other antimicrobial substances.

**Claims for the Contracting State: AT**

1. Process for preparing primycin salts the primycin component of which is unchanged in its structure and antibiotic activity from the primycin obtained by fermentation, characterised in that a suspension of primycin sulphate in an aliphatic $C_{1-4}$ alcohol is reacted with a barium salt of an organic acid or an inorganic acid and the resulting primycin salt is isolated.

2. Process according to claim 1, characterised in that a barium salt of an aliphatic $C_{1-4}$ or $C_{14-16}$ carboxylic acid, a halogenated aliphatic carboxylic acid, an aliphatic dicarboxylic acid, an aromatic carboxylic acid, a substituted aromatic carboxylic acid, an organic sulphonic acid or a halic acid is used in the reaction.

3. Process according to claim 2, characterised in that the barium salt used is barium formate, acetate, monochloroacetate, trichloroacetate, propionate, palmitate, oxalate, perchlorate, bromide, iodide, benzoate, mesylate or tosylate.

4. Process according to claims 1 to 3, characterised in that the barium salt used is one which is formed *in situ*.

5. Process according to claims 1 to 4, characterised in that, in order to prepare *in situ* the barium salts used, barium carbonate or a solution thereof in an aliphatic $C_{1-4}$ alcohol is dissolved in the corresponding organic or inorganic acid, the resulting solution is concentrated by evaporation and the residue is dissolved in an aliphatic $C_{1-4}$ alcohol or in ether.

6. Process according to claims 1 to 5, characterised in that, in order to prepare *in situ* the barium salts used, an aqueous solution of barium hydroxide is reacted with a solution of corresponding organic or inorganic acid in an aliphatic $C_{1-4}$ alcohol and/or water and then filtered off.

7. Process according to claims 1 to 6, characterised in that the reaction of primycin sulphate with a barium salt is carried out at a temperature in the range from 20 to 80°C, preferably at the boiling temperature of the solvent.

8. Process according to claims 1 to 7, characterised in that, in order to isolate the primycin salt, the barium sulphate is filtered off, the filtrate is concentrated by evaporation *in vacuo* and the residue is triturated with a solvent, preferably with a mixture of acetone and water (2:1) and/or ether.

9. Use of one or more of the primycin salts obtainable according to the processes of claims 1 to 8, optionally together with other antibacterially active substances, as active substances for producing pharmaceutical preparations with an antibiotic activity.